# EUROPEAN PATENT APPLICATION

(11) **EP 4 082 569 A1**
(43) Date of publication of application: **02.11.2022**
(21) Application number: 21171614.7
(22) Date of filing: 30.04.2021
(51) Int. Cl.: A61K 39/39, A61P 37/02

(54) **COLLAGEN HYDROLYSATES AS VACCINE ADJUVANTS**

(71) Applicant: DSM IP Assets B.V., 6411 TE Heerlen (NL)
(72) Inventor: GIESSER, Joanne, 4303 Kaiseraugst (CH); HUG, Hubert Paul, 4303 Kaiseraugst (CH); KUENZLI, Katharina, 4303 Kaiseraugst (CH); MLADIC, Marija, 4303 Kaiseraugst (CH); VAN DER HOEVEN, Robertus Antonius Mijndert, 4303 Kaiseraugst (CH); WYSS, Adrian, 4303 Kaiseraugst (CH)
(74) Representative: Kurt, Manfred

(57) **Abstract**

It has been found that collagen hydrolysates have pro-inflammatory activities, and thus can be used as vaccine adjuvants.

## Description

### BRIEF DESCRIPTION OF THE INVENTION

We tested effects of collagen peptides and hydrolysates on LPS-induced pro-inflammatory cytokines in an assay with BEAS-2B cells, a human lung cell line. Cytokines measured were IL-6, IL-8, MCP-1. The peptides did not show any significant effect on the secretion of inflammatory cytokines. The hydrolysates, on the other hand, showed an increase in the three tested inflammatory markers. Thus the collagen hydrolysates are pro-inflammatory action and can be used as adjuvants in vaccines.

### BACKGROUND OF THE INVENTION

An adjuvant is an ingredient used in some vaccines that helps create a stronger immune response in people receiving the vaccine. In other words, adjuvants help vaccines work better. Some vaccines that are made from weakened or killed bacteria or viruses contain naturally occurring adjuvants and help the body produce a strong protective immune response. However, most vaccines developed today include just small components of bacteria or viruses, such as a proteins. Adjuvants help the body to produce an immune response strong enough to protect the person from the disease he or she is being vaccinated against. Adjuvanted vaccines can cause more local reactions (such as redness, swelling, and pain at the injection site) and more systemic reactions (such as fever, chills and body aches) than non-adjuvanted vaccines.

Adjuvants have been used safely in vaccines for decades.

Aluminum salts, such as aluminum hydroxide, aluminum phosphate, and aluminum potassium sulfate have been used safely in vaccines for more than 70 years. Aluminum salts were initially used in the 1930s, 1940s, and 1950s with diphtheria and tetanus vaccines after it was found they strengthened the body's immune response to these vaccines.

Newer adjuvants have been developed to target specific components of the body's immune response, so that protection against disease is stronger and lasts longer.

| **Adjuvant** | **Composition** | **Vaccines** |
|---|---|---|
| **Aluminum** | One or more of the following: amorphous aluminum hydroxyphosphate sulfate (AAHS), aluminum hydroxide, aluminum phosphate, potassium aluminum sulfate (Alum) | Anthrax, DT, DTaP (Daptacel), DTaP (Infanrix), DTaP-IPV (Kinrix), DTaP-IPV (Quadracel), DTaP-HepB-IPV (Pediarix), DTaP -IPV/Hib (Pentacel), Hep A (Havrix), Hep A (Vaqta), Hep B (Engerix-B), Hep B (Recombivax), HepA/Hep B (Twinrix), HIB (PedvaxHIB), HPV (Gardasil 9), Japanese encephalitis (Ixiaro), MenB (Bexsero, Trumenba), Pneumococcal (Prevnar 13), Td (Tenivac), Td (Mass Biologics), Tdap (Adacel), Tdap (Boostrix) |
| AS04 | Monophosphoryl lipid A (MPL) + aluminum salt | Cervarix |
| MF59 | Oil in water emulsion composed of squalene | Fluad |
| AS01_{B} | Monophosphoryl lipid A (MPL) and QS-21, a natural compound extracted from the Chilean soapbark tree, combined in a liposomal formulation | Shingrix |
| CpG 1018 | Cytosine phosphoguanine (CpG), a synthetic form of DNA that mimics bacterial and viral genetic material | Heplisav-B |
| No adjuvant | | ActHIB, chickenpox, live zoster (Zostavax), measles, mumps & rubella (MMR), meningococcal (Menactra, Menveo), rotavirus, seasonal influenza (except Fluad), single antigen polio (IPOL), yellow fever |

### Aluminum

Aluminum-containing adjuvants are vaccine ingredients that have been used in vaccines since the 1930s. Small amounts of aluminum are added to help the body build stronger immunity against the virus or bacteria in the vaccine.

### AS04

Beginning in 2009, monophosphoryl lipid A (MPL) was used in one U.S. vaccine (Cervarix^{®}); however, the vaccine is no longer available in the United States due to low market demand. This immune-boosting substance was isolated from the surface of bacteria.

### MF59

MF59 is the adjuvant contained in Fluad (an influenza vaccine licensed for adults aged 65 or older). MF59 is an oil-in-water emulsion composed of squalene, which is a naturally occurring oil found in many plant and animal cells, as well as in humans. MF59, has been used in flu vaccines in Europe since 1997 and in the United States since 2016.

### AS01 B

AS01 B is an adjuvant suspension used with the antigen component of Shingrix vaccine. Shingrix is the recombinant zoster vaccine recommended for persons aged 50 years or older. AS01 B is made of up of monophosphoryl lipid A (MPL), an immune-boosting substance isolated from the surface of bacteria, and QS-21, a natural compound extracted from the Chilean soapbark tree (*Quillaja saponaria* Molina

### CpG 1018

CpG 1018 is a recently developed adjuvant used in Heplisav-B vaccine. It is made up of cytosine phosphoguanine (CpG) motifs, which is a synthetic form of DNA that mimics bacterial and viral genetic material. When CpG 1018is included in a vaccine, it increases the body's immune response.

Inflammation is a key process of the innate immune system to activate an active immune response. Thereby, Interleukin-8 (IL-8) is part of the innate immune system and important in the initiation of an immune response. Interleukin-6 (IL-6) is secreted by T-lymphocytes and macrophages and helps also to stimulate an immune response. Monocyte chemoattractant protein-1 (MCP-1) recruits monocytes, memory T-lymphocytes and dendritic cells to the site of inflammation.

It would be desirable to have a new vaccine adjuvant which is derived from a natural product.

### DETAILED DESCRIPTION OF THE INVENTION

We tested effects of collagen peptides and hydrolysates on LPS-induced pro-inflammatory cytokines in an assay with BEAS-2B cells, a human lung cell line. Cytokines measured were: IL-6, IL-8, and MCP-1. Four collagen-derived peptides were tested in the assay, along with the commercial preparations of collagen hydrolysates (bovine or fish), and also enzymatic hydrolysates of the commercial preparations. Surprisingly, the peptides were not active, however the hydrolysates and the further hydrolyzed preparations were. Thus, the collagen hydrolysates can be used as adjuvants in vaccines.

While not wishing to be bound by theory, we believe that the difference in activity is due to the presence of glycosylated or other sugar moieties on the peptides, as the non-glycosylated peptides were not active, but the hydrolysates were. The various receptors involved in generating innate immune responses, including the TOLL receptors, are known to interact with glycosylation sites. Further, the activity did not depend on the type of collagen, as both bovine and fish collagen was found to be pro-inflammatory. Further, the size of the hydrolysates did not seem to be a critical factor, as both the hydrolysates which were commercially obtained, and the subsequently treated enzyme-treated hydrolysates were also active.

Thus one embodiment of this invention is a vaccine adjuvant comprising a pro-inflammatory amount of a collagen hydrolysate.

### BRIEF DESCRIPTION OF THE FIGURES

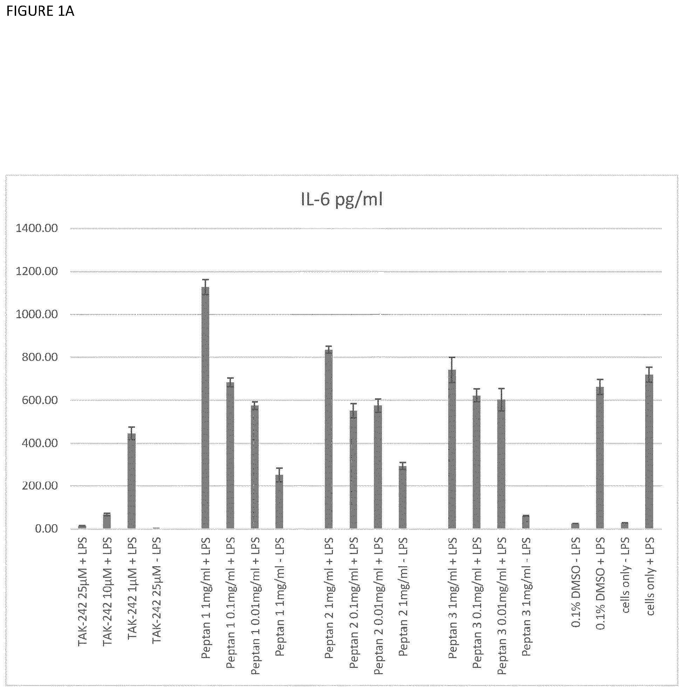

**Figure 1A****:** IL-6 secretion (pg/ml) of BEAS-2B cells pretreated for 2 h with the indicated concentrations of bovine collagen hydrolysates peptan 1, peptan 2, and peptan 3 in a final DMSO solvent concentration of 0.1 %, followed by a simulation of inflammation with 200 ng/ml LPS and overnight incubation. Positive control TAK-242, is given at the left, LPS-controls are at the right. The experiment was done in triplicate.

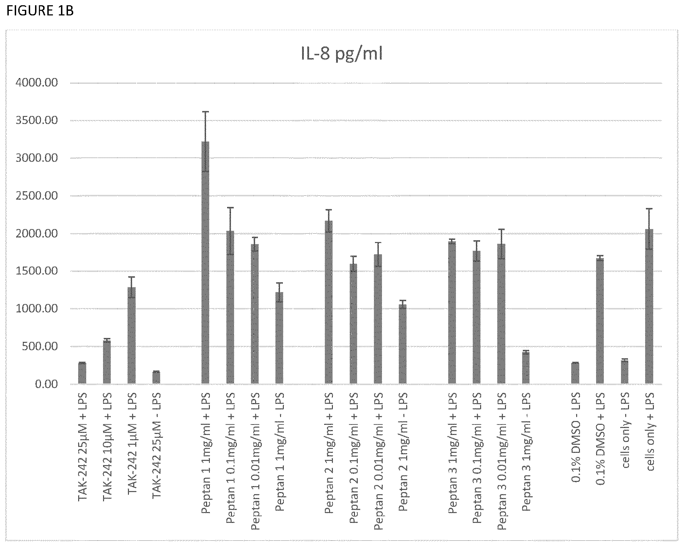

**Figure 1B****:** IL-8 secretion (pg/ml) of BEAS-2B cells pretreated for 2 h with the indicated concentrations of bovine collagen hydrolysates peptan 1, peptan 2, and peptan 3 in a final DMSO solvent concentration of 0.1 %, followed by a simulation of inflammation with 200 ng/ml LPS and overnight incubation. Positive control TAK-242, is given at the left, LPS-controls are at the right.

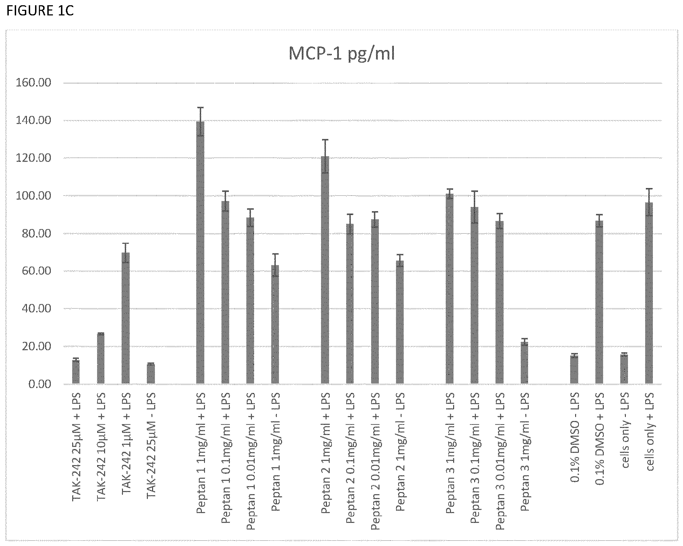

**Figure 1C****:** MCP-1 secretion (pg/ml) of BEAS-2B cells pretreated for 2 h with the indicated concentrations of bovine collagen hydrolysates peptan 1, peptan 2, and peptan 3 in a final DMSO solvent concentration of 0.1 %, followed by a simulation of inflammation with 200 ng/ml LPS and overnight incubation. Positive control TAK-242, is given at the left, LPS-controls are at the right.

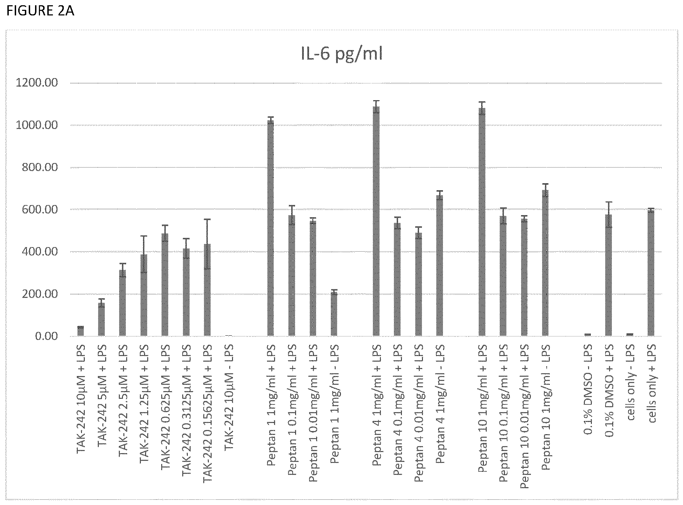

**Figure 2A****:** IL-6 secretion (pg/ml) of BEAS-2B cells pretreated for 2 h with the indicated concentrations of bovine collagen hydrolysates peptan 1, peptan 4, and peptan 10 in a final DMSO solvent concentration of 0.1 %, followed by a simulation of inflammation with 200 ng/ml LPS and overnight incubation. The positive control TAK-242 is given at the left, LPS-controls are at the right.

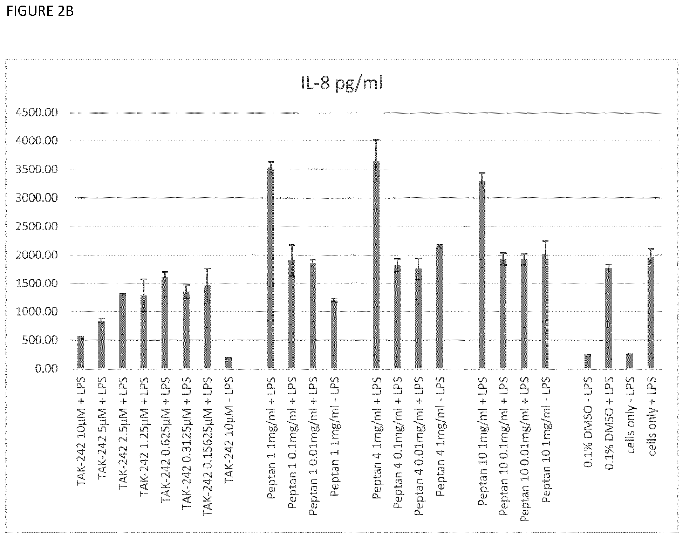

**Figure 2B****:** IL-8 secretion (pg/ml) of BEAS-2B cells pretreated for 2 h with the indicated concentrations of bovine collagen hydrolysates peptan 1, peptan 4, and peptan 10 in a final DMSO solvent concentration of 0.1 %, followed by a simulation of inflammation with 200 ng/ml LPS and overnight incubation. The positive control TAK-242 is given at the left, LPS-controls are at the right.

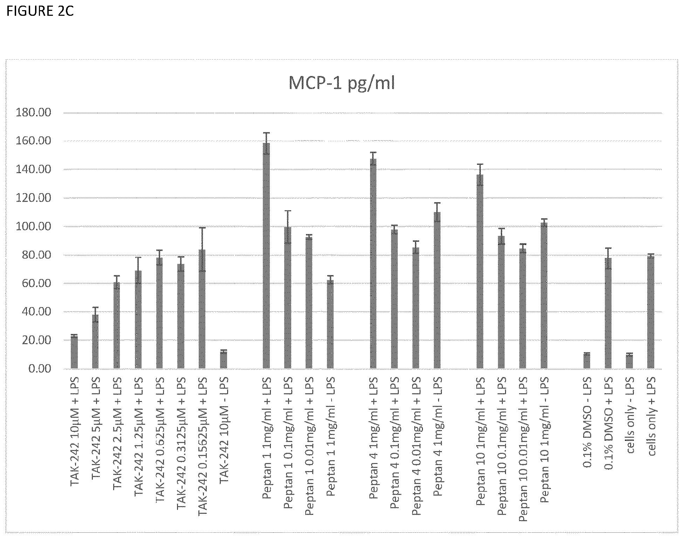

**Figure 2C****:** MCP-1 secretion (pg/ml) of BEAS-2B cells pretreated for 2 h with the indicated concentrations of bovine collagen hydrolysates peptan 1, peptan 4, and peptan 10 in a final DMSO solvent concentration of 0.1 %, followed by a simulation of inflammation with 200 ng/ml LPS and overnight incubation. The positive control TAK-242 is given at the left, LPS-controls are at the right.

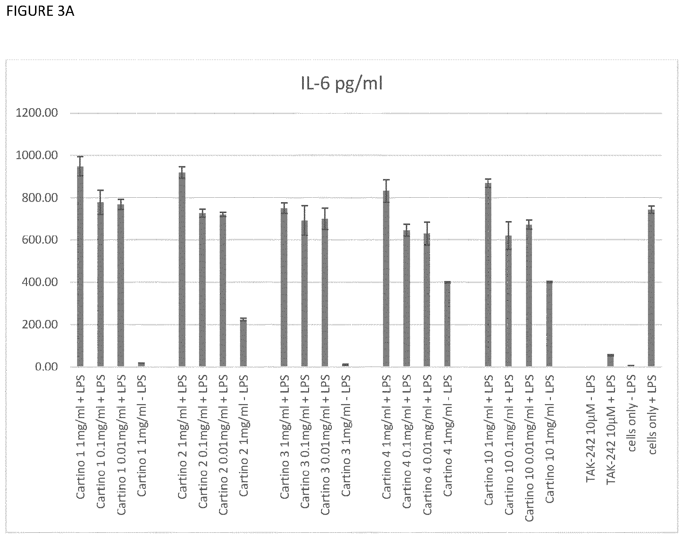

**Figure 3A****:** IL-6 secretion (pg/ml) of BEAS-2B cells pretreated for 2 h with the indicated concentrations of fish collagen hydrolysates cartino 1, cartino 2, cartino 3, and cartino 4 in a final DMSO solvent concentration of 0.1 %, followed by a simulation of inflammation with 200 ng/ml LPS and overnight incubation. The positive control TAK-242 and the LPS-control are at the right.

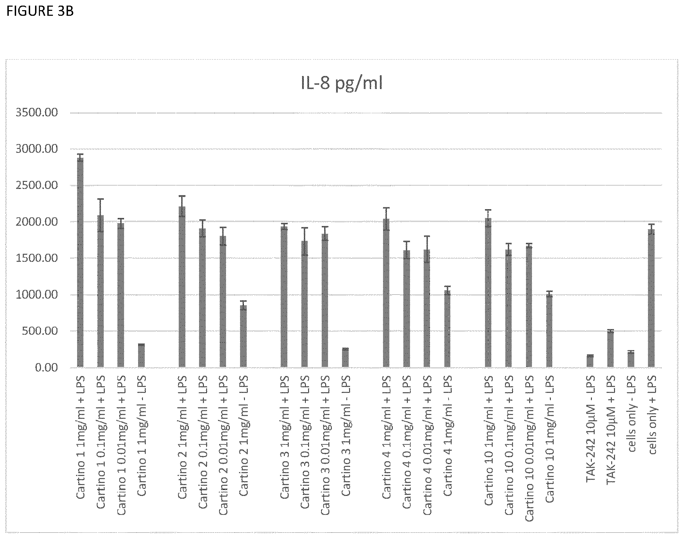

**Figure 3B****:** IL-8 secretion (pg/ml) of BEAS-2B cells pretreated for 2 h with the indicated concentrations of fish collagen hydrolysates cartino 1, cartino 2, cartino 3, and cartino 4 in a final DMSO solvent concentration of 0.1 %, followed by a simulation of inflammation with 200 ng/ml LPS and overnight incubation. The positive control TAK-242 and the LPS-control are at the right.

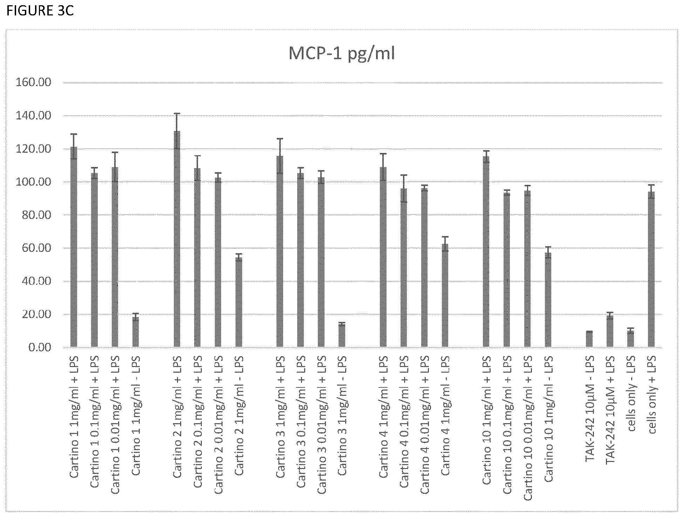

**Figure 3C****:** MCP-1 secretion (pg/ml) of BEAS-2B cells pretreated for 2 h with the indicated concentrations of fish collagen hydrolysates cartino 1, cartino 2, cartino 3, and cartino 4 in a final DMSO solvent concentration of 0.1 %, followed by a simulation of inflammation with 200 ng/ml LPS and overnight incubation. The positive control TAK-242 and the LPS-control are at the right.

In some embodiments is may be helpful to reduce the size of the commercially available collagen by subjecting it to a size-reduction treatment. The treatment may be selected from known procedures such as subjecting the protein/peptide to mechanical shear force, ultrasound treatment, or enzyme treatment. In some preferred embodiments, an enzyme treatment is used.

Thus, another embodiment of this invention is a composition comprising a collagen hydrolysate which has undergone further enzymatic treatment. In some embodiments, the collagen hydrolysate is of bovine or fish origin. Bovine or fish collagens are often sold in hydrolyzed form for use in the food industry are readily available from commercial sources.

In another embodiment, the hydrolysate is made by treating commercially available collagen with at least one an enzyme, preferably selected from the group consisting of: a papain, a cysteine protease, or combinations of an endoprotease and a proline specific endoprotease, while retaining the sites which have sugar residues.

As the size of the resultant peptide fractions does not seem to be critical, the enzymatic treatment is typically for 1-6 hours, preferably for about two hours.

Thus another embodiment of this invention is a collagen enzymatic hydrolysate made by a process comprising subjecting a collagen hydrolysate to an enzyme treatment, so that the resulting enzymatic hydrolysate is characterized as being pro-inflammatory.

The hydrolysates of this invention can be used as a vaccine adjuvant. They can help activate the immune system, and particularly help to stimulate innate immunity to allow the vaccine antigen to be more antigenic, and thus improve the performance of the vaccine. Thus another embodiment of this invention is a vaccine comprising an antigenic agent in combination with an enzymatic collagen hydrolysate. Preferably the collagen hydrolysate is a bovine or fish collagen hydrolysate.
A further embodiment is a method of activation of the innate immune system in a person at risk of suffering from an infectious disease comprising administering a composition comprising a collagen hydrolysate to a person in need thereof.

### Usage and formulation

Since collagen hydrolysate will be digested to small peptides and amino acids, the method of application is injection or co-injection. The amount of adjuvant in the vaccine is usually quite low, for example under 2 mg per dose of vaccine. In preferred amounts the adjuvant of this invention is present in amounts from 0.1 mg to 0.75 mg per dose, but this can vary due to the nature of the vaccine antigen.

The following non-limiting Examples are presented to further illustrate the invention.

### EXAMPLES

### EXAMPLE 1

### Materials and Methods

The human bronchial epithelial cell line BEAS-2B was from ATCC (American Type Culture Collection, Manassas, VA, ATCC CRL-9609) and cultured in Bronchial Epithelial cell Growth Medium (BEGM, CC-3170, Lonza, Wakersville, MD) in CellBIND^{®} surface plastic flasks (Corning Inc., Corning, NY). The cells were cultured at 37 °C in a humidified atmosphere containing 5 % CO₂.

BEAS-2B cells were seeded in 12-well CellBIND^{®} surface culture plates (Corning Inc.) at 3 to 5 × 10⁵ cells per well. Cells were treated with 900 µl diluted with collagen hydrolysates or peptides for 2 hrs, the 100 µl 10 x LPS was added, and incubated overnight. Final Concentrations of peptides were: 10 µM, 2 µM, 400 nM, 80 nM. The final DSMO concentrations were 0.1%. Untreated cells or cells treated with 0.1 % DMSO were used as controls. After 24 h, cell supernatants were collected.

The concentrations of IL-6, IL-8, and MCP-1 in the supernatants were determined by Luminex kits (BIO-RAD Laboratories, Hercules, CA) and used in the LiquiChip Workstation IS 200 (Qiagen, Hilden, Germany). The data were evaluated with the LiquiChip Analyser software (Qiagen).

Cell survival assays of the BEAS-2B and A549 cells were performed with PrestoBlue Cell Viability Reagent Kit (Lubioscience) according to the protocol of the manufacturer. Non-toxic concentrations of the peptides, collagen hydrolysates and single compounds were selected for the assays.

### Positive controls

TAK-242 (Resatorvid, CAS 243984-11-4) (5 mg from Sigma (614316), Lot 3523494) was used as positive control for the assay. Stock solution was 25 mM in DMSO, and final concentrations were: 25 µM, 10 µM, 1 µM. This is a cell-permeable compound that selectively binds to Cys747 of TLR4 and selectively disrupts its interaction with adaptor molecules TIRAP and TRAM (Ono, Y., Maejima, Y., Saito, M. et al. TAK-242, a specific inhibitor of Toll-like receptor 4 signaling, prevents endotoxemia-induced skeletal muscle wasting in mice. Sci Rep 10, 694 (2020).

### Inducer of inflammation

Lipopolysaccharides (LPS) from Escherichia coli (O55:B5, Sigma L6529) ware used to induce inflammation. The stock solution was 500 µg/ml BEGM, the final concentration on cells 200 ng/ml.

Starting material:
- Hydrolyzed Bovine collagen (Peptan B 5000 LD, Peptan); https://www.ulprospector.com/en/na/Food/Detail/1694/195077/Peptan-B-5000-HD, also containing the datasheet
- Hydrolyzed Fish collagen (Car2006, Cartino) http://www.cartinogelatin.co.th/cartinogelatin/cartinocollagen.html

### Hydrolysates

**Table 1: Collagen hydrolysates. Protease treatments are indicated.**

| | | | | | | | |
|---|---|---|---|---|---|---|---|
| **Collagen Hydrolysates:** | | | | | | | |

| Collagen | Number | Protease name | pH incubation | Temp incubation, C | Dose enzyme (%w/w DM)* | Time, h | Enzyme form |
|---|---|---|---|---|---|---|---|
| Peptan (Bovine) | 1 | No enzyme | 5.5 | 45 | - | 2 | |
| | 2 | Collupulin (papain) | 5.5 | 45 | 0.1 | 2 | liquid |
| | 3 | Denazyme PMC soft (collagenase - Nagase) | 5.5 | 45 | 0.5** | 2 | powder |
| | 4 | Maxipro FPC | 5.5 | 45 | 0.1 | 2 | powder |
| | 5 | Maxipro NPU | 5.5 | 45 | 0.1 | 2 | liquid |
| | 6 | Maxipro BAP | 5.5 | 45 | 0.1 | 2 | liquid |
| | 7 | Maxipro PSP | 5.5 | 45 | 0.1 | 2 | liquid |
| | 8 | Maxipro NPU + Maxipro PSP | 5.5 | 45 | 0.1+0.1 | 2 | Liquid |
| | 9 | Denazyme PMC +Maxipro PSP | 5.5 | 45 | 0.1+0.1 | 2 | Liquid |
| | 10 | Maxipro FPC +Maxipro PSP | 5.5 | 45 | 0.1+0.1 | 2 | Powder /liquid |
| | 11 | Maxipro PSP + XPP-015 (Nagase) | 5.5 | 45 | 0.1+0.1 | 2 | Liquid / powder |
| Cartino (Fish) | 1 | No enzyme | 5.5 | 45 | - | 2 | |
| | 2 | Collupulin (papain) | 5.5 | 45 | 0.1 | 2 | liquid |
| | 3 | Denazyme PMC soft (collagenase - Nagase) | 5.5 | 45 | 0.5** | 2 | powder |
| | 4 | Maxipro FPC | 5.5 | 45 | 0.1 | 2 | powder |
| | 5 | Maxipro NPU | 5.5 | 45 | 0.1 | 2 | liquid |
| | 6 | Maxipro BAP | 5.5 | 45 | 0.1 | 2 | liquid |
| | 7 | Maxipro PSP | 5.5 | 45 | 0.1 | 2 | liquid |
| | 8 | Maxipro NPU + Maxipro PSP | 5.5 | 45 | 0.1+0.1 | 2 | Liquid |
| | 9 | Denazyme PMC +Maxipro PSP | 5.5 | 45 | 0.1+0.1 | 2 | Liquid |
| | 10 | Maxipro FPC +Maxipro PSP | 5.5 | 45 | 0.1+0.1 | 2 | Powder /liquid |
| | 11 | Maxipro PSP + XPP-015 (Nagase) | 5.5 | 45 | 0.1+0.1 | 2 | Liquid / powder |

Preparations Bovine 4-9 were not tested in the immunoassay; Preparations Fish 4-9 were also not yet tested in the assay.

Enzymes used in Table 1 above are as follows:
Collupulin (papain)(Sigma Aldrich) is a cysteine protease of the papain family. Papain exhibits proteolytic activity against amide links, amino acid esters, peptides, proteins. It mainly cleaves peptide bonds containing amino acids such as lysine, arginine and residues succeeding phenylalanine
Denazyme PMC (Nagase) is a pure, microbial protease that selectively hydrolyzes connective tissue (the triple-helical protein called collagen).
Maxipro FPC (Protease A form Amano), fungal aspecific endoprotease from *Aspergillus oryzae*
MAxipro NPU (Neutral Protease), (Sigma Aldrich) A serine endopeptidase that consists primarily of subtilisin A from *Bacillus Lichenifornis,* a specific endoprotease.
Maxipro PSP, proline specific protease from *Aspergillus Niger* (DSM), Cleaves specifically at the C-terminus of Proilin, endoprotease.

We tested the effect of different collagen hydrolysates on the secretion of anti-inflammatory cytokines. The test system were BEAS-2B cells treated with LPS. The used collagen hydrolysates and their treatments are summarized in Table 1, above.

For all hydrolysates and peptides viability was determined (data not shown) as describes in the methods section and based on these result concentrations were selected.

Upon stimulation with LPS, BEAS-2B cells secreted the pro-inflammatory cytokines IL-6, IL-8, and MCP-1. Therefore, we concentrated our measurements on these three cytokines.
Unexpectedly all tested collagen hydrolysates did not show a decrease of the concentration of the cytokines, but rather an increase **(****Figures 1 - 3****).** Therefore, collagen hydrolysates are pro-inflammatory in our cell-culture system.
For bovine collagen hydrolysates results are shown in **Figures 2** **and** **3****.** LPS induced a strong secretion of all three cytokines which was not significantly changed in the presence of 0.1 % DMSO. A strong inhibitor of LPS-induced secretion of pro-inflammatory cytokines is the TLR4-antagonist TAK-242, used as control.

Bovine Peptan 1 and peptan 2 showed an increase of IL-6 **(****Figure 1A****),** IL-8 **(****Figure 1B**), and MCP-1 **(****Figure 1C****)** secretion even in the absence of LPS. In the presence of LPS, cytokine secretion **(****Figure 1A-C****)** was further enhanced, especially with peptan 1. The induction of IL-6, IL-8. And MCP-1 cytokine secretion was weaker by peptan 3 **(****Figure 1A-C****).**

Similarly, the effects of bovine peptan4 and peptan 10 are shown in **Figure 2****.** Here, the positive control was TAK-242 from 0.16 µM to 10 µM. Both, peptan 4 and peptan 10, showed effects on cytokine secretion even stronger to peptan 1 **(****Figure 2A-C****).** Peptan 4 and peptan 10 induced the secretion of the pro-inflammatory cytokines IL-6 **(****Figure 2A****),** IL-8 **(****Figure 2B****),** and MCP-1 **(****Figure 2C****)** in the same range as LPS.

Similar results were obtained with fish collagen hydrolysates **(****Figure 3****).** Cartino 2, cartino 4, and cartino 10 showed a strong induction of IL-6 **(****Figure 1A****),** IL-8 **(****Figure 1B**), and MCP-1 **(****Figure 1C****)** secretion, again in the absence of LPS. In these cases, the levels reached were blow those obtained with LPS **(****Figure 3****).** For cartino 1 and cartino 3 no IL-6 induction was detected **(****Figure 3A****).** Induction of secretion of IL-8 **(****Figure 3B****)** and MCP-1 (Figure 3C) was weak for cartino 1 and cartino 3.

In contrast to the results with collagen hydrolysates, collagen-derived peptides used at concentrations of 50 - 100 µM had no effect on LPS-induced secretion of cytokines IL-6 and IL-8 in BEAS-2B cells (data not shown). MCP-1 was not measured. Therefore, we speculate that the sugar moieties present in the hydrolysates are responsible for the pro-inflammatory effect.

## Claims

1. A vaccine adjuvant comprising a pro-inflammatory amount of a collagen hydrolysate.

2. An adjuvant according to claim 1 which is bovine or fish collagen.

3. An adjuvant according to Claim 1 or 2 which is a collagen hydrolysate which has undergone further enzymatic treatment.

4. An adjuvant according to any of Claims 1-3 **characterized in that** the hydrolysate is made by treating commercially available collagen with at least one an enzyme, preferably selected from the group consisting of: a papain, a cysteine protease, or combinations of an endoprotease and a proline specific endoprotease, while retaining the sites which have sugar residues.

5. An adjuvant according to Claim 3 or 4, wherein the enzymatic treatment is typically for 1-6 hours, preferably for about two hours.

6. A collagen enzymatic hydrolysate made by a process comprising subjecting a collagen hydrolysate to a further enzyme treatment, and the resulting enzymatic hydrolysate is characterized as being pro-inflammatory.

7. A method of activating a person's immune system comprising administering an antigenic agent in combination with an enzymatic collagen hydrolysate.

8. A method according to claim 7 wherein the innate immune system is activated.

9. A method according to claim 7 or 8 wherein the collagen hydrolysate is a bovine or fish collagen hydrolysate.

10. A method according to any of Claims 7-9 wherein the person is at risk of suffering from an infectious disease.
